# EUROPEAN PATENT SPECIFICATION

(11) **EP 1 065 199 B1**
(45) Date of publication and mention of the grant of the patent: **19.01.2005**
(21) Application number: 00111609.4
(22) Date of filing: 31.05.2000
(51) Int. Cl.: C07C 303/44, C07C 309/65

(54) **Process for the recovery of perfluorinated acids from aqueous solution**
Verfahren zur Gewinnung von Perfluorsäuren aus wässriger Lösung
Procédé de récupération d'acides perfluorés de solution aqueuse

(30) Priority: 30.06.1999 DK 93799
(43) Date of publication of application: 03.01.2001
(73) Proprietor: Haldor Topsoe A/S, 2800 Lyngby (DK)
(72) Inventor: Hommeltoft, Sven Ivar, 3400 Hillerod (DK)
(74) Representative: Patentanwälte Zellentin & Partner

(56) References cited:
- EP-A- 0 614 699
- EP-A- 0 671 213
- EP-A- 0 687 658
- EP-A- 1 044 963

## Description

The present invention relates to recovery of strong acid from an aqueous solution. In particular, the invention provides a process for the recovery of those acids in anhydrous form from the aqueous substrate.

Fluorinated and perfluorinated sulphonic acids are known as valuable catalysts in various reactions including alkylation of hydrocarbons (US Patent Nos. 5,220,095, 5,245,100 and US 5,498,820). These processes typically produce small amounts of spent acid, in which the sulphonic acid catalyst is inactive with respect to the desired reactions. In order to ensure feasible economics, the acid catalyst has to be recovered from the spent acid and recycled in its anhydrous form to the alkylation process.

A method for the recovery of fluorinated and perfluorinated sulphonic acid from spent acid by use of sulphuric acid is disclosed in EP 614,699. Use of esters of sulphuric acid in the recovery of the acid from spent acid is furthermore disclosed in US Patent No. 5,472,921. It is additionally known to extract the acid from spent acid with water, and, subsequently, recover the acid from the aqueous solution. Recovery of anhydrous acid from the aqueous solution may be achieved by distillation of a salt of the acid with sulphuric acid as mentioned in US Patent No. 5,618,769.

By another method to isolate the acid in its anhydrous form, the acid is isolated from aqueous solution by addition of a weak base in form of a salt of the acid to the aqueous solution of the acid in order to facilitate the separation of the acid and the water. Thereby, it is possible to remove substantially all water from the mixture in a first evaporation step and subsequently recover the anhydrous acid in a second evaporation step as described in US Patent Nos. 5,603,812 and 5,759,357. As an advantage of the above additive distillation, the additive can be recycled to the process and the process does not consume chemicals and does not produce waste chemicals. The disadvantage of this process is that evaporation at reduced pressure conditions (approx. 25-50 mbar) is required in order not to decompose the acid. The rate of decomposition of the acid increases with temperatur.

Thus, the general object of the present invention is to improve the known distillation process by means of a stripping agent.

Accordingly, this invention is a process for the recovery of an acid in its anhydrous form, comprising the steps of:
a) providing an aqueous solution with the acid in its hydrated form;
b) adding to the solution a weak base in form of a soluble salt of the acid;
c) concentrating the solution of the acid and the acid salt to a substantially water free acid-salt mixture by action of a stripping agent; and
d) subjecting the acid-salt mixture to distillation obtaining the acid in its anhydrous form.

Optionally the aqueous solution can be concentrated prior to step b).

When practising the invention with an inert stripping agent such as n-alkane, the evaporation step can be performed in a distillation column. The stripping agent vapour assists in allowing evaporation at a pressure close to or at atmospheric pressure compared to the close to vacuum pressure in known processes, by taking up the differential partial pressure. Whilst increasing the total pressure, the partial pressures of the components to be separated are maintained, thereby maintaining an allowable operation temperature. An additional advantage of the invention is that heat may be introduced into the process by means of the stripping agent. Thereby, direct heating of the aqueous acidic and potentially corrosive solutions are advantageously avoided and employment of expensive materials in the heat transfer surfaces made superfluous.

The invention will be described more detailed in the following Examples.

### EXAMPLES

### Example 1

Stripping of water at atmospheric pressure from a mixture of water, trifluoromethanesulphonic acid and triethylammonium triflate using n-hexane at 200-250°C.

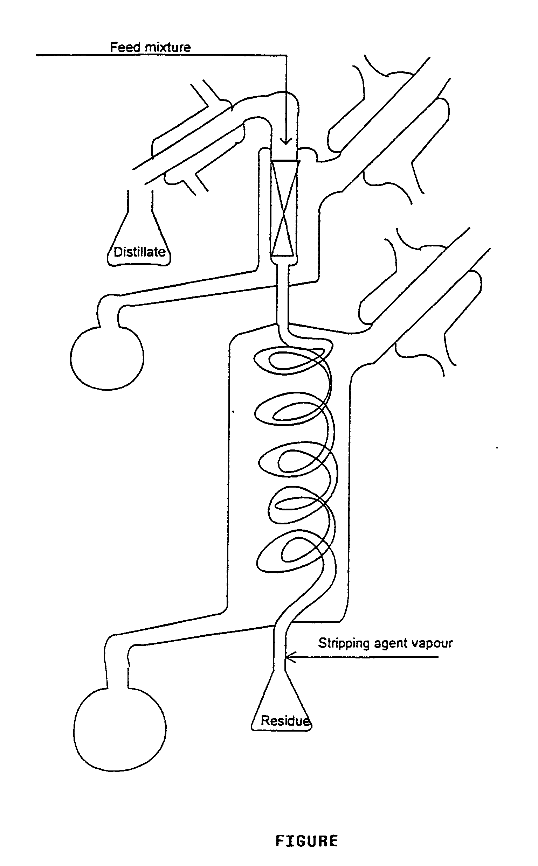

The equipment used in this experiment is illustrated in the attached Figure.

The lower part of the apparatus is shown in the Figure consists of a glass spiral (ID=10 mm) heated on the outside by condensing tetradecane to a temperature of about 252°C. A stripping agent (hexane, approximately 6g/min.) was evaporated and preheated before being introduced at bottom of the heated glass spiral. The residual mixture was withdrawn below the glass spiral. A distillation column (ID=3cm, height=28 cm, packed with 4 mm Wilson helices) is arranged on top of the glass spiral. The column was heated to 252°C on the outside of the column by condensing tetradecane. A feed mixture of 20 wt% trifluoromethanesulphonic acid, 42 wt% triethylammonium triflate and 38 wt% water was introduced at a rate of 11-12 g/min at top of the distillation column operating at atmospheric pressure. The stripping agent and stripped off product were withdrawn at a point above the distillation column. Upon condensation this stream separated into an aqueous phase containing small amounts of the acid and a hydrocarbon phase containing the stripping agent.

The aqueous phase withdrawn together with the stripping agent at top of the distillation column contained 4 wt% trifluoromethanesulphonic acid. The residual acid-salt mixture withdrawn at bottom of the glass spiral contained 30 wt% trifluoromethanesulphonic acid and less than 0.1 wt% water.

The feed mixture containing 0.47g trifluoromethanesulphonic acid and 0.90g water per gram triethyl ammonium triflate was concentrated to an anhydrous mixture containing 0.43g trifluoromethanesulphonic acid per gram triethylammonium triflate.

The acid-salt mixture was subjected to distillation as conventionally obtaining the acid in its anhydrous form.

### Example 2

Stripping of water and acid at atmospheric pressure from a mixture of water, trifluoromethanesulphonic acid and triethyl ammonium triflate using hexane at 200-250°C.

The experiment was performed by means of the above described equipment and by using a similar procedure and the same feed mixture composition as in Example 1, except that the flow of the water/acid/salt mixture was 3 g/min. Consequently, most of the acid as well as all the water was stripped off the salt. The aqueous phase of the overhead product contained 30.5 wt% trifluoromethanesulphonic acid and the residual salt mixture contained 6 wt% acid and no water.

The same feed mixture composition as in Example 1 was concentrated to an anhydrous mixture containing 0.07g trifluoromethanesulphonic acid per g salt by using a lower feed/stripping agent ratio.

Example 2 demonstrates that the separation at atmospheric pressure of acid from the acid salt by means of a stripping agent is possible.

## Claims

1. Process for the recovery of a perfluorinated acid in its anhydrous form, comprising the steps of:
a) providing an aqueous solution with the acid in its hydrated form;
b) adding to the solution a weak base in form of a soluble salt of the acid;
c) concentrating the solution of the acid and the acid salt to a substantially water free acid-salt mixture by distillation in the presence of a first stripping agent; and
d) subjecting the acid-salt mixture to distillation optionally in the presence of a second stripping agent in order to obtain a salt remanence and the acid in its anhydrous form and further optionally recirculating the salt remanence from this distillation step to process step b).

2. Process according to claim 1, wherein the salt of the acid comprises a salt of the acid with an organic base.

3. Process according to claim 2, wherein the organic base is selected from trialkyl amines.

4. Process according to claim 1, wherein the acid is a perfluoroalkane sulphonic acid.

5. Process according to claim 4, wherein the fluorinated sulphonic acid comprises trifluoromethane sulphonic acid.

6. Process according to claim 1, wherein the first and/or the second stripping agent is a condensable hydrocarbon.

7. Process according to claim 6, wherein the pressure is kept sufficiently high to allow for condensation of the stripping agent.

8. Use of a process according to any of the preceding claims for the recovery of a perfluorinated sulphonic acid from the effluent stream of an alkylation process catalysed by the acid.

9. Use of a process according to claim 8, wherein the effluent from the alkylation process is subjected to a first removal of water e.g. by means of distillation, prior to adding the soluble salt of the acid catalyst.

## Patentansprüche

1. Verfahren zur Gewinnung einer perfluorierten Säure in ihrer wasserfreien Form umfassend die Schritte:
a) Bereitstellung einer wäßrigen Lösung mit der Säure in ihrer hydratisierten Form;
b) Zufügung einer schwachen Base in Form eines löslichen Salzes der Säure zu der Lösung;
c) Aufkonzentrieren der Lösung der Säure und des Säuresalzes zu einer im wesentlichen wasserfreien Säure-Salz-Mischung durch Destillation in Gegenwart eines ersten Strippmittels; und
d) Destillieren der Säure-Salz-Mischung gegebenenfalls in Gegenwart eines zweiten Strippmittels, um einen Salzrückstand und die Säure in ihrer wasserfreien Form zu erhalten und gegebenenfalls weiterhin Rückführung des Salzrückstands aus diesem Destillationsschritt in den Verfahrensschritt b).

2. Verfahren gemäß Anspruch 1, **dadurch gekennzeichnet, daß** das Salz der Säure ein Salz der Säure mit einer organischen Base umfaßt.

3. Verfahren gemäß Anspruch 2, **dadurch gekennzeichnet, daß** die organische Base ausgewählt ist aus Trialkylaminen.

4. Verfahren gemäß Anspruch 1, **dadurch gekennzeichnet, daß** die Säure eine Perfluoralkansulfonsäure ist.

5. Verfahren gemäß Anspruch 4, **dadurch gekennzeichnet, daß** die fluorierte Sulfonsäure Trifluormethansulfonsäure umfaßt.

6. Verfahren gemäß Anspruch 1, **dadurch gekennzeichnet, daß** das erste und/oder das zweite Strippmittel ein kondensierbarer Kohlenwasserstoff ist.

7. Verfahren gemäß Anspruch 6, **dadurch gekennzeichnet, daß** der Druck ausreichend hoch eingestellt wird, um eine Kondensation des Strippmittels zu erlauben.

8. Verwendung eines Verfahrens gemäß einem der vorhergehenden Ansprüche zur Gewinnung einer Perfluorsulfonsäure aus dem Ablaufstrom eines Alkylierungsverfahrens, welches durch die Säure katalysiert wird.

9. Verwendung eines Verfahrens gemäß Anspruch 8, **dadurch gekennzeichnet, daß** der Ablauf aus dem Alkylierungsverfahren zuerst einer Entfernung von Wasser unterworfen wird, z.B. mittels Destillation, bevor das lösliche Salz der Katalysatorsäure zugefügt wird.

## Revendications

1. Procédé pour récupérer un acide perfluoré sous sa forme anhydre, comprenant les étapes de :
a) fourniture d'une solution aqueuse avec l'acide sous sa forme hydratée ;
b) addition d'une base faible à la solution sous la forme d'un sel soluble de l'acide ;
c) concentration de la solution de l'acide et du sel de l'acide en un mélange acide/sel sensiblement sans eau par distillation en présence d'un premier agent de stripage ; et
d) soumission du mélange acide/sel à une distillation, optionnellement en présence d'un deuxième agent de stripage, de façon à obtenir un sel de rémanence et l'acide sous sa forme anhydre et en outre optionnellement recycler le sel de rémanence à partir de l'étape de distillation pour le processus de l'étape b).

2. Procédé selon la revendication 1, dans lequel le sel de l'acide comprend un sel de l'acide avec une base organique.

3. Procédé selon la revendication 2, dans lequel on choisit la base organique parmi les amines trialkylées.

4. Procédé selon la revendication 1, dans lequel l'acide est un acide perfluoroalcanesulfonique.

5. Procédé selon la revendication 4, dans lequel l'acide sulfonique f luoré comprend l'acide trifluorométhanesulfonique.

6. Procédé selon la revendication 1, dans lequel le premier et/ou le deuxième agent de stripage est un hydrocarbure condensable.

7. Procédé selon la revendication 6, dans lequel la pression est maintenue à une valeur suffisamment élevée pour permettre la condensation de l'agent de stripage.

8. Utilisation d'un procédé selon l'une quelconque des revendications précédentes pour la récupération d'un acide sulfonique perfluoré à partir du courant d'effluent d'un processus d'alkylation catalysé par l'acide.

9. Utilisation d'un procédé selon la revendication 8, dans lequel on soumet l'effluent du processus d'alkylation à une première suppression d'eau par exemple au moyen d'une distillation, avant d'ajouter le sel soluble du catalyseur acide.
